# EUROPÄISCHE PATENTSCHRIFT

(11) **EP 2 478 832 B1**
(45) Veröffentlichungstag und Bekanntmachung des Hinweises auf die Patenterteilung: **20.09.2017**
(21) Anmeldenummer: 12150156.3
(22) Anmeldetag: 04.01.2012
(51) Int. Cl.: A61B 3/13, G02B 7/00, G02B 7/02, G02B 21/00

(54) **Positioniereinheit und ophtalmologisches Mikroskop**
Positioning unit and ophtalmologic microscope
Unité de positionnement et microscope ophtalmologique

(30) Priorität: 20.01.2011 DE 102011002941
(43) Veröffentlichungstag der Anmeldung: 25.07.2012
(73) Patentinhaber: Oculus Optikgeräte GmbH, 35582 Wetzlar-Dutenhofen (DE)
(72) Erfinder: Feiertag, Carsten, 35410 Hungen (DE); Pfeiffer, Günter, 35075 Gladenbach (DE); Kirchhübel, Rainer, 35614 Asslar (DE)
(74) Vertreter: advotec.

(56) Entgegenhaltungen:
- DE-A1- 3 822 008
- DE-A1- 4 116 810
- DE-A1-102008 011 608
- DE-U1- 20 215 635

## Beschreibung

Die Erfindung betrifft ein Mikroskop mit einer Positioniereinheit zur Positionierung einer zumindest eine Ophthalmoskopierlinse umfassenden optischen Einheit in einem Strahlengang eines Mikroskops zwischen einem Objektiv des Mikroskops und vor einem zu beobachtenden Auge, wobei die Positioniereinheit eine Anschlussvorrichtung umfasst, mittels der die Positioniereinheit an das Mikroskop koppelbar ist, wobei die Positioniereinheit eine Positioniervorrichtung umfasst, mittels der das optische Element relativ zum Mikroskop in Längsrichtung des Strahlengangs bewegbar ist. Mikroskope zur Durchführung von Augenoperationen werden regelmäßig für Operationen in einem vorderen Bereich eines Auges verwendet. Sollen auch in einem hinteren Bereich eines Auges derartige Eingriffe vorgenommen werden, ist es notwendig das Mikroskop mit einer Beobachtungsvorrichtung zu ergänzen, welche eine Fokussierung eben dieses Bereiches des Auges ermöglicht. Derartige Beobachtungsvorrichtungen umfassen zumindest eine Weitwinkellinse bzw. Ophthalmoskopierlinse zur Weitwinkelbetrachtung des betreffenden hinteren Teil des Auges, wobei die Ophthalmoskopierlinse ein Zwischenbild in einem Strahlengang vor einem Objektiv des Mikroskops zur Verfügung stellt. Dieses Zwischenbild kann mit dem Mikroskop nicht fokussiert werden. Je nach Brennweite der Zusatzoptik und des betrachteten Auges erscheint das Zwischenbild an einer Position scharf, die näher zum Objekt hin liegt. Zur Fokussierung des Zwischenbildes bedarf es einer Verkürzung der Brennweite des Mikroskopobjektives. Eine Höhenverstellung des Mikroskops ändert die Brennweite nicht. Durch Einsatz einer Reduzierlinse im Strahlengang unter dem Mikroskopobjektiv lässt sich die Zwischenbildebene in den Fokus des Mikroskops legen. Um dieses Zwischenbild mit dem Mikroskop fokussieren zu können, muss das Mikroskop um eine Strecke relativ zur Ophthalmoskopierlinse bewegt bzw. entfernt werden. Diese Höhenveränderung wird wesentlich durch die individuelle Brechkraft des Auges und durch die unterschiedliche Brechkraft der gewählten Ophthalmoskopierlinse bestimmt.

Die beiden Linsen werden von einer Positioniereinheit der Beobachtungsvorrichtung, welche unmittelbar am Mikroskop befestigt ist, gehaltert und können je nach Bedarf im Strahlengang positioniert werden, ohne dass es einer wesentlichen Anpassung des Mikroskops während einer Operation bedarf. Die Positioniereinheit umfasst regelmäßig eine Anschlussvorrichtung, mittels der die Positioniereinheit an das Mikroskop koppelbar ist. Weiter ist die Positioniereinheit so ausgebildet, dass die betreffenden Linsen einfach in den Strahlengang eingeschwenkt bzw. eingeschoben und wieder daraus entfernt werden können.

Um eine möglichst genaue Anpassung des Zwischenbildes der Ophthalmoskopierlinse an eine Brennweite des Mikroskopobjektivs vornehmen zu können, kann zumindest eine der Linsen längs des Strahlengangs des Mikroskops einstellbar ausgebildet sein. Bei bekannten Beobachtungsvorrichtungen ist zur längsverschiebbaren Einstellung der Linse beispielsweise an der Positioniereinheit eine lineare Führung ausgebildet, wobei die Linse mittels eines Einstellrades mit einem Gewindetrieb bewegt werden kann. Um während einer Operation eines Auge eine versehentliche Kollision der Ophthalmoskopierlinse mit dem Auge zu verhindern bzw. eine mögliche Augenverletzung zu vermeiden, ist die Positioniereinheit so ausgebildet, dass die Ophthalmoskopierlinse in Richtung des Objektivs des Mikroskops im wesentlichen widerstandslos bewegbar ist, dass heißt bei einer Kollision mit dem Auge zurückweichen kann. Dies wird beispielsweise durch eine zweite lineare Führung erreicht, die ebenfalls eine Längsverschiebung der Ophthalmoskopierlinse ermöglicht.

Neben den zuvor beschriebenen, mechanischen und optischen Anforderungen ist es wichtig, dass die Beobachtungsvorrichtung bzw. die Positioniereinheit bei einer Operation im Wesentlichen steril ist, um eine mögliche Infektion eines Auges mit beispielsweise Keimen zu verhindern. Eine Infektionsgefahr besteht insbesondere dadurch, dass die Beobachtungsvorrichtung bei einer Operation relativ dicht an das betreffende Auge herangeführt wird. Die Möglichkeit einer Infektion des Patientenauges durch eine unzureichende aufbereitete Positioniereinheit wird durch Verwendung einer steril gelieferten Einmal-Positioniereinheit ausgeschlossen. Es ist daher üblich, die betreffende Beobachtungsvorrichtung bzw. Positioniereinheit vor einer Operation durch beispielsweise Dampfsterilisation zu sterilisieren. Um eine wiederholte Sterilisation durchführen zu können, ist es zwingend notwendig alle Bauteile der Beobachtungsvorrichtung bzw. Positioniereinheit, mit Ausnahme eventuell vorhandener Dichtungen aus elastischen Materialien wie Gummi, aus Metall oder Glas auszubilden. Andere Werkstoffe, wie beispielsweise Kunststoffe, haben sich für eine wiederholte Sterilisation als nicht sehr haltbar erwiesen. Auch die Linearführungen und der Gewindetrieb bedürfen zur Sicherstellung bestimmter Passungen einer maßhaltigen Ausbildung, sodass auch hier nur Bauteile aus Metall in Frage kommen. Um ein Eindringen von Wasser bei der Sterilisation in die Führungen zu verhindern, können diese mit Gummidichtungen oder Dichtungen aus anderen elastischen Materialien versehen sein. Weiter ist es notwendig entsprechende Gleitflächenpaarungen der Führungen bzw. des Gewindetriebs in regelmäßigen Abständen mit einem Schmiermittel zu schmieren, um deren Funktion sicher zu stellen.

Die aus dem Stand der Technik bekannten Beobachtungsvorrichtungen bzw. Positioniereinheiten weisen eine Reihe von Nachteilen auf. So ist ein Gewicht der Beobachtungsvorrichtung, welche beispielweise an einer am Mikroskop verschraubten Adapterplatte an das Mikroskop adaptiert werden kann, relativ hoch und bei einer Handhabung der Beobachtungsvorrichtung bei einer Operation störend. Auch sind die linearen Führungen nur schwer abzudichten oder zu sterilisieren. In die Gewindegänge des Gewindetriebs kann bei einer Dampfsterilisation Wasser bzw. Dampf nur schwer eindringen, sodass nach der Dampfsterilisation sich noch unerwünschte Wasserreste oder Keime in den Gewindegängen befinden können. Weiter ist es störend, dass die verwendeten Schmiermittel bei der Dampfsterilisation zumindest teilweise entfernt werden und das zur Sterilisation verwendete Wasser verunreinigen. Auch die Sterilisation selbst ist als problematisch anzusehen, da nicht mit absoluter Sicherheit ausgeschlossen werden kann, dass sich nach der Dampfsterilisation noch Keime an der Beobachtungsvorrichtung bzw. Positioniereinheit befinden. So ist eine Qualität einer Sterilisation unter anderem auch abhängig von einer Wasserqualität in einem Dampfsterilisationsgerät.

Weiter ist eine Beobachtungsvorrichtung bzw. eine Positioniereinheit nach jeder Verwendung zu sterilisieren, sodass die Beobachtungsvorrichtung bzw. Positioniereinheit nicht bei direkt der Verwendung nachfolgenden Augenoperationen aufgrund der Sterilisationszeiten eingesetzt werden kann. So ist es gegebenenfalls notwendig immer mehrere Beobachtungsvorrichtungen bzw. Positioniereinheiten zu beschaffen und vorzuhalten, damit ohne zeitliche Einschränkung operiert werden kann. So ergeben sich insgesamt hohe Kosten für eine Herstellung, Sterilisation und Wartung der Beobachtungsvorrichtung bzw. Positioniereinheit, sowie Kosten durch eine erhöhte Kapitalbindung für einen Anwender.

Die DE 10 2008 011 608 A1 zeigt eine Positioniereinheit für ein ophthalmologisches Operationsmikroskop, wobei die Positioniereinheit zwei schwenkbare, mit einem Gelenk verbundene Arme aufweist. An einem Ende eines Arms ist eine drehbare Aufnahmeeinrichtung angeordnet, an der zwei Ophthalmoskopierlinsen mittels eines Halters befestigt werden können.

Aus der DE 202 15 635 U1 ist eine Positioniereinheit für eine Ophthalmoskopierlinse bekannt, bei der die Ophthalmoskopierlinse mittels eines motorisch angetriebenen Schneckengetriebes in vertikaler Richtung relativ zu einem Mikroskop in einer Längsrichtung eines Strahlengangs bewegbar ist.

Die DE 41 16 810 A1 beschreibt eine Positioniereinheit für ein Endoskop, welches an einem Operationsmikroskop adaptiert werden kann. Die Positioniereinheit umfasst ein scherenartiges Gelenkstück, mittels dem das Endoskop relativ zum Mikroskop in Längsrichtung verschoben werden kann. Das scherenartige Gelenkstück ist an einem Ende drehbar an einem Halter, und an einem gegenüberliegenden Ende in einer Längsnut in dem Halter verschiebbar gelagert.

Der vorliegenden Erfindung liegt daher die Aufgabe zugrunde, eine Positioniereinheit und eine Beobachtungsvorrichtung vorzuschlagen, die einen leichter zu reinigenden Bewegungsmechanismus aufweist.

Diese Aufgabe wird durch ein Mikroskop mit den Merkmalen des Anspruchs 1 gelöst.

Die Positioniereinheit zur Positionierung einer zumindest ein optisches Element umfassenden optischen Einheit in einem Strahlengang eines Mikroskops zwischen einem Objektiv des Mikroskops und vor einem zu beobachtenden Auge umfasst eine Anschlussvorrichtung, mittels der die Positioniereinheit an das Mikroskop koppelbar ist, wobei die Positioniereinheit eine Positioniervorrichtung umfasst, mittels der das optische Element relativ zum Mikroskop in Längsrichtung des Strahlengangs bewegbar ist, wobei die Positioniervorrichtung aus einem ersten Doppelschwingengetriebe und einem zweiten Doppelschwingengetriebe gebildet ist, wobei die Doppelschwingengetriebe mittels eines gemeinsamen Koppelgliedes miteinander verbunden sind.

Eine Bewegbarkeit des optischen Elements in Längsrichtung des Strahlengangs ermöglicht eine Anpassung der optischen Einheit an das zu beobachtende Auge und/oder eine Anpassung des Strahlengangs des Mikroskops an ein im Strahlengang befindliches Zwischenbild, ohne dass diesbezügliche Einstellungen am Mikroskop vorgenommen werden müssen. Die Doppelschwingengetriebe sind vorzugsweise aus jeweils zwei Schwingen gebildet, die an ihren Enden ihrerseits jeweils mit einem Drehlager verbunden sind. So wird eine kreisbogenförmige Bewegung der ersten Doppelschwinge mit dem Koppelglied ermöglicht, wobei die zweite Doppelschwinge eine ebenfalls kreisbogenförmige Bewegung in die gleiche Richtung ausführen kann, derart, dass sich aus beiden kreisförmigen Bewegungen eine lineare Bewegung in Längsrichtung des Strahlengangs ergibt. Durch diese Kombination zweier Doppelschwingengetriebe kann vollständig auf eine Linearführung zur Bewegung des optischen Elements verzichtet werden. Die Doppelschwingengetriebe sind mit einfachen Drehgelenken oder Drehlagern ausbildbar, welche gegenüber Linearführungen wesentlich einfacher zu sterilisieren bzw. abzudichten sind. Auch bedarf es keiner besonderen Wartung oder Schmierung der Doppelschwingengetriebe sowie der Ausbildung spezieller Führungen mit entsprechend engen Toleranzen. Die Schwingen der Doppelschwingengetriebe sowie das Koppelglied können in diesem Fall beispielsweise auch kostengünstig aus Metall hergestellt werden. Insgesamt kann so ein Herstellungsaufwand für die Positioniereinheit wesentlich verringert werden.

Wenn die Positioniereinheit zumindest teilweise aus Kunststoffmaterial gebildet ist, lassen sich die Herstellungskosten für die Positioniereinheit erheblich verringern. So können wesentliche, für eine mechanische Funktion zwingend erforderliche Bauteile der Positioniereinheit, beispielsweise in einem Spritzgussverfahren, kostengünstig hergestellt werden. Die dann durch die Verwendung von Kunststoffmaterial erzielbare Kosteneinsparung ermöglicht es auf eine Wiederverwendung der Positioniereinheit gänzlich zu verzichten und die Positioniereinheit nach einer Benutzung zu entsorgen. Dadurch ergeben sich weitere Kostenvorteile, da keine Kosten für eine Aufbereitung und Wartung anfallen. Durch die so mögliche Einmalverwendung der Positioniereinheit können weiter auch mit der Sterilisation verbundene Verschmutzungsrisiken und mögliche Defekte an der Positioniereinheit ausgeschlossen werden. So können möglichst viele Bauteile der Positioniereinheit bzw. kostenintensiv herzustellende Bauteile aus einem Kunststoffmaterial ausgebildet sein. Die Positioniereinheit kann somit in Art eines sterilen Einmalartikels, welcher in einer Schutzverpackung geliefert werden kann, ausgebildet sein. Da dann an eine Wiederverwertung oder Sterilisation der Positioniereinheit nicht mehr gedacht werden muss, kann ein besonders kostengünstiges Kunststoffmaterial verwendet werden. Eine derart ausgebildete Positioniereinheit kann insbesondere auch dann eingesetzt werden, wenn aufgrund besonderer Hygienevorschriften eine Verwendung resterilisierter Instrumente untersagt ist. Weiter ergeben sich dann für Augenoperationen keine Wartezeiten durch in einer Sterilisation befindliche Instrumente.

Erfindungsgemäß ist, um eine sichere Führung des optischen Elements in Längsrichtung des Strahlengangs zu gewährleisten, das erste Doppelschwingengetriebe mit dem zweiten Doppelschwingengetriebe über ein Zahnradgetriebe verbunden, derart, dass eine Bewegung des ersten Doppelschwingengetriebes auf das zweite Doppelschwingengetriebe mittels des Zahnredgetriebes übertragbar ist. Zur Bildung des Zahnradgetriebes kann es schon ausreichend sein, wenn zumindest ein Zahn eines der Doppelschwingengetriebe mit einem Zahnpaar des anderen Doppelschwingengetriebes in Eingriff steht. Die jeweiligen Zähne können in Verlängerung einer Schwinge der Doppelschwingengetriebe ausgebildet sein. So kann das Zahnradgetriebe und somit eine Bewegungskopplung der beiden Doppelschwingengetriebe besonders einfach und kostengünstig ausgebildet werden. Wenn die Schwingen der Doppelschwingengetriebe aus Kunststoff gebildet sind, kann das Zahnradgetriebe einfach an den jeweiligen Schwingen angeformt sein.

Insbesondere beim Einsatz eines Spritzgussverfahrens zur Herstellung der Doppelschwingengetriebe können die Doppelschwingengetriebe und das Zahnradgetriebe zusammen einstückig ausgebildet sein. Das Zahnradgetriebe kann beispielsweise zwischen zwei Schwingen, welche die jeweils notwendigen Zähne ausbilden, gebildet werden. Die Zähne können an den Schwingen angeformt oder durch eine Verlängerung von Enden der Schwingen ausgebildet sein. Eine einstückige Ausbildung kann insbesondere dadurch erleichtert werden, dass die Doppelschwingengetriebe und das Zahnradgetriebe im Wesentlichen in einer zweidimensionalen Ebene ausgebildet sind. Eine Herstellung derartiger Kunststoffteile in einem Spritzgussverfahren ist besonders einfach möglich.

Besonders vorteilhaft ist es, wenn das Zahnradgetriebe ein Übersetzungsverhältnis von 1:1 aufweist. So kann bei im Wesentlichen übereinstimmenden Längen der Doppelschwingengetriebe eine gleichförmige und übereinstimmende Bewegung derselben ermöglicht werden, die eine lineare Relativbewegung des optischen Elements in Längsrichtung des Strahlengangs sicherstellt.

Weiter kann eine erste Doppelschwinge des ersten Doppelschwingengetriebes an einem Anschlusslagerglied und eine zweite Doppelschwinge des zweiten Doppelschwingengetriebes an einem Aufnahmelagerglied gelagert sein. Das Anschlusslagerglied und das Aufnahmelagerglied können so jeweils die beiden Schwingen der Doppelschwingengetriebe in einem definierten Abstand miteinander verbinden. So kann das Anschlusslagerglied für eine starre Befestigung im Bereich des Mikroskops vorgesehen sein, wobei das Koppelglied relativ zum Anschlusslagerglied und das Aufnahmelagerglied relativ zum Koppelglied und zum Anschlusslagerglied bewegbar ist. Im Bereich des Aufnahmelagergliedes kann demnach das optische Element bzw. die optische Einheit vorgesehen sein. Sowohl das Anschlusslagerglied als auch das Aufnahmelagerglied kann wie die Doppelschwingen und das Koppelglied aus einem Kunststoffmaterial gebildet sein.

Um das optische Element relativ längs des Strahlengangs bewegen zu können, kann die Positioniervorrichtung eine Einstelleinrichtung umfassen, mittels der eine Position des optischen Elements einstellbar ist. So kann sichergestellt werden, dass sich das optische Element in der jeweils gewünschten Position befindet, wobei die Einstellung bzw. Positionierung des optischen Elements beispielsweise manuell durch eine Bedienperson erfolgen kann.

Die Einstelleinrichtung kann beispielsweise aus zumindest einem Einstellrad mit einem Schnecken- oder Exzentergetriebe gebildet sein. So kann das Einstellrad in einer Ausführungsform am Anschlusslagerglied gelagert sein und mittels einer am Einstellrad angeformten Schnecke auf eine Schwinge des ersten oder zweiten Doppelschwingengetriebes einwirken. Eine Drehung des Einstellrads erwirkt dann eine Abstandsänderung der Schwinge relativ zum Einstellrad je nachdem welcher Bereich der Schnecke mit der Schwinge im Eingriff steht. Aus der daraus resultierenden Bewegung der Schwinge ergibt sich folglich eine Bewegung beider Doppelschwingengetriebe und somit eine Längsbewegung des optischen Elements. Eine derartige Einstelleinrichtung kann auch besonders einfach aus einem Kunststoffmaterial hergestellt werden. Beispielsweise kann das Einstellrad mit der Schnecke als ein Spritzgussteil gefertigt werden, welches einfach auf eine Nabe aufsteckbar ist. Um eine beidseitige Bedienung zu ermöglichen, können auch zwei gegenüberliegend angeordnete Einstellräder vorgesehen werden.

Zum Schutz eines Auges durch eine unbeabsichtigte Kollision mit dem optischen Element kann die Positioniervorrichtung eine Sicherheitsrichtung ausbilden, die eine lose Bewegung des optischen Elements ermöglicht, wenn auf das optische Element eine Kraft in Richtung des Mikroskops ausgeübt wird. Das heißt, die Positioniervorrichtung bzw. Sicherheitseinrichtung kann so ausgebildet sein, dass bei einer Krafteinwirkung auf das optische Element, beispielsweise hervorgerufen durch eine Kollision mit dem betreffenden Auge, dass optische Element im Wesentlichen widerstandslos in Richtung des Objektivs bewegt werden kann. So kann die Sicherheitseinrichtung so ausgebildet sein, dass die Positioniervorrichtung und die optische Einheit durch ihr jeweiliges Eigengewicht, das optische Element in einer unteren Position in Nähe des Auges hält. Wird dann in Richtung des Mikroskops eine Kraft auf das optische Element aufgebracht, ist lediglich eine Gewichtskraft der optischen Einheit bzw. Positioniervorrichtung zu überwinden, um das optische Element bewegen zu können. Insbesondere durch die Verwendung der Doppelschwingengetriebe kann so gegenüber einer Linearführung ein Verklemmen der Sicherheitseinrichtung verhindert werden. Weiter ist bei einer Verwendung von Kunststoffmaterial für die betreffenden Bauteile eine Gewichtskraft derselben vergleichsweise gering, sodass nur eine geringe Kraft zur Bewegung des optischen Elements aufgewandt werden muss. Sofern die Gewichtskraft so weit vermindert ist, dass auch eine unerwünschte Bewegung des optischen Elements nicht mehr ausgeschlossen werden kann, kann zu dessen Stabilisierung eine Feder an der Positioniervorrichtung vorgesehen werden, die eine zusätzliche Kraft in Richtung des Auges aufbringt.

Vorteilhaft kann die Positioniereinheit eine Wechselvorrichtung umfassen, mittels der das optische Element in den Strahlengang hinein und hinaus bewegbar ist. Die Wechselvorrichtung kann so ausgebildet sein, dass das optische Element in den Strahlengang einschiebbar oder einschwenkbar ist. Vorzugsweise kann das optische Element um eine quer zum Strahlengang verlaufende Achse zusammen mit der Positioniereinheit verschwenkt werden. So kann sichergestellt werden, dass die Positioniereinheit und die optische Einheit während einer Operation eine Sicht in einem Bewegungsraum des Operierenden oberhalb des betreffenden Auges nicht einschränkt bzw. behindert. Auch wird es so möglich, das optische Element je nach Bedarf einfach in den Strahlengang hinein und heraus zu bewegen.

Die Wechselvorrichtung kann durch die Anschlussvorrichtung und die Positioniervorrichtung ausgebildet sein, derart, dass die Positioniervorrichtung relativ zur Anschlussvorrichtung verschwenkbar ist. Hilfsweise kann die Positioniervorrichtung unmittelbar so mit der Anschlussvorrichtung verbunden sein, dass sie relativ zur Anschlussvorrichtung bewegt bzw. verschwenkt werden kann. Zur Ausbildung einer derartigen Wechselvorrichtung werden nicht notwendigerweise zusätzliche Bauteile benötigt.

Auch kann die Anschlussvorrichtung ebenfalls aus einem Kunststoffmaterial gebildet sein. Die Anschlussvorrichtung kann unmittelbar mit dem Mikroskop fest verbindbar ausgebildet sein oder alternativ mit einer Adaptereinrichtung, die ihrerseits fest mit dem Mikroskop verbunden ist. In diesem Fall kann die Anschlussvorrichtung ohne zu Hilfenahme von zusätzlichem Werkzeug mit der Adaptereinrichtung, beispielsweise in Art einer Steckverbindung, verbunden werden. Sofern eine Adaptereinrichtung am Mikroskop vorgesehen ist, kann diese selbstverständlich auch aus einem Kunststoffmaterial gebildet sein.

Die Wechselvorrichtung ist besonders einfach herstellbar, wenn die Wechselvorrichtung als ein Drehgelenk ausgebildet ist, wobei die Wechselvorrichtung zumindest eine Rasteinrichtung umfassen kann, mittels der das optische Element in einer Verwendungsposition im Strahlengang und/oder in einer Nichtverwendungsposition außerhalb des Strahlengangs adaptierbar ist. Das Drehgelenk kann zwischen der Anschlussvorrichtung und der Positioniervorrichtung ausgebildet sein, sodass ein Verschwenken der Positioniervorrichtung relativ zur Anschlussvorrichtung möglich ist. Weiter kann an der Anschlussvorrichtung und der Positioniervorrichtung die Rasteinrichtung ausgebildet sein, welche ihrerseits aus einer Rastnase und Rastvertiefungen zum Eingriff der Rastnase ausgebildet sein kann. Die Rastnase und die Rastvertiefungen können jeweils an der Anschlussvorrichtung oder der Positioniervorrichtung angeformt sein. Die Rastvertiefungen können dann so angeordnet sein, dass die Rastnase in der Verwendungsposition und der Nichtverwendungsposition jeweils in eine Rastvertiefung eingreift und so eine Arretierung des optischen Elements bzw. der Positioniervorrichtung ermöglicht.

Drehlager bzw. Schwingenlager der Doppelschwingengetriebe können besonders einfach durch jeweils ein Filmscharnier ausgebildet sein. Insbesondere, wenn die Doppelschwingengetriebe vollständig aus Kunststoff ausgebildet sind ist dies besonders vorteilhaft, da so sämtliche Bauteile der Doppelschwingengetriebe in einem Spritzgussverfahren zusammen mit den daran angeformten Schwingenlagern hergestellt werden können. Weiter kann so auch auf die sonst erforderliche Montage der Doppelschwingengetriebe verzichtet werden.

Die Positioniereinheit ist besonders kostengünstig und einfach herstellbar, wenn die Positioniereinheit vollständig aus Kunststoffmaterial ausgebildet ist. Die Positioniereinheit kann dann aus nur wenigen Bauteilen gebildet sein, da es dann möglich ist, in beispielsweise einem Kunststoffspritzgussverfahren auch räumlich komplexe Bauteile herzustellen, die aus Metall nur mit hohem Aufwand herstellbar wären. Als ein Kunststoffmaterial kann beispielsweise, insbesondere aufgrund seiner mechanischen Eigenschaften, ein Polyamid verwendet werden. Auch wird es so möglich, die gesamte Positioniereinheit nach einer Benutzung ohne weiteren Aufwand einem kontaminierten Sondermüll zuzuführen.

Die erfindungsgemäße Beobachtungsvorrichtung umfasst eine erfindungsgemäße Positioniereinheit und zumindest eine optische Einheit, wobei die optische Einheit zumindest ein optisches Element umfasst. Die optische Einheit ist somit Bestandteil der Beobachtungsvorrichtung, welche in alternativen Ausführungsformen mehrere optische Einheiten aufweisen kann. Auch ist vorgesehen, das die optische Einheit zumindest ein optisches Element, wie beispielsweise eine Linse oder ein Prisma aufweist, wobei auch mehrere optische Elemente vorgesehen sein können, die eine Gruppe von Linsen oder Prismen der optischen Einheit bilden. Hinsichtlich der Vorteile der erfindungsgemäßen Beobachtungsvorrichtung wird auf die vorangehenden Merkmalsbeschreibungen der Positioniereinheit verwiesen.

Um eine leicht handhabbare Verbindung zwischen Positioniereinheit und optischer Einheit auszubilden, kann die Positioniereinheit eine Aufnahmevorrichtung umfassen, mittels der die optische Einheit an der Positioniereinheit adaptierbar ist, wobei die optische Einheit eine Haltereinrichtung zur Halterung und Verbindung des optischen Elements mit der Aufnahmevorrichtung ausbilden kann. Die Aufnahmevorrichtung ermöglicht es, die optische Einheit von der Positioniereinheit getrennt auszubilden und je nach Bedarf die optische Einheit, während beispielsweise einer Augenoperation auszuwechseln, ohne die gesamte Positioniereinheit austauschen zu müssen. Auch bleibt es einem Operierenden dann selbst überlassen, die Positioniereinheit mit der optischen Einheit bedarfsgerecht zur Beobachtungsvorrichtung zu ergänzen. Um eine standardisierte Verbindung der optischen Einheit mit der Aufnahmevorrichtung zu ermöglichen, kann das oder die optischen Elemente von der Halteeinrichtung in der vorgesehenen Position gehaltert werden, wobei die Halteeinrichtung in Verbindung mit der Aufnahmevorrichtung der Positioniereinheit, beispielsweise in Art einer Steckverbindung, ausgebildet sein kann. Durch diese Schnittstelle an der Positioniereinheit, können auch konventionell ausgebildete, optische Einheiten an die Positioniereinheit adaptiert werden.

Alternativ kann die Positioniereinheit eine Halteeinrichtung zur Halterung des optischen Elements ausbilden. Folglich kann das optische Element unmittelbar von der Positioniereinheit gehaltert werden, ohne dass die Ausbildung einer Aufnahmevorrichtung an der Positioniereinheit notwendig wäre. Insbesondere wenn die Positioniereinheit aus Kunststoffmaterial gebildet ist, kann die Halteinrichtung an der Positioniereinrichtung angeformt sein, sodass für eine Montage des optischen Elements dieses lediglich in die Halteeinrichtung eingesetzt werden muss.

Um eine einmalige Benutzung der Beobachtungsvorrichtung sicherzustellen, kann die Halteeinrichtung und/oder die Aufnahmevorrichtung und/oder eine Anschlussvorrichtung zumindest ein Verbindungselement aufweisen, welches so ausgebildet ist, dass es bei einem Trennen von Halteeinrichtung und Aufnahmevorrichtung und/oder Anschlussvorrichtung und einem Mikroskop zerstört wird. Insbesondere, wenn zur Ausbildung der Beobachtungsvorrichtung Bauteile aus Kunststoff verwendet werden, ist eine Sterilisation der Beobachtungsvorrichtung bzw. der Kunststoffbauteile nicht möglich und auch nicht erwünscht. Folglich ist sicherzustellen, dass diese Bauteile nicht bei weiteren Augenoperationen wiederverwendet werden. An der Halteeinrichtung, der Aufnahmevorrichtung oder der Anschlussvorrichtung kann demnach ein Verbindungselement vorgesehen sein, welches beispielsweise in Art eines Rastelements mit einer Sollbruchstelle ausgebildet ist und bei einer Montage der Bauteile so verrastet, sodass eine Demontage nur mit einer zwangläufigen Zerstörung des Verbindungselements möglich ist. Eine erneute Montage und Verwendung wird so erschwert bzw. unmöglich gemacht. Weiter kann das zerstörte Verbindungselement bzw. das betreffende Bauteil von einem Benutzer als bereits verwendet und somit unbrauchbar erkannt werden.

Auch die Beobachtungsvorrichtung wird noch kostengünstiger herstellbar, wenn die optische Einheit aus Kunststoffmaterial gebildet ist. Auch können ein oder mehrere optische Elemente der optischen Einheit aus Kunststoffmaterial mit entsprechender optischer Qualität hergestellt sein.

Die optische Einheit kann auch einstückig ausgebildet sein, insbesondere wenn die Halteeinrichtung aus dem gleichen Material wie das optische Element ausgebildet ist. Das optische Element kann dann zusammen mit der Halteeinrichtung in einem Spritzguss- oder Pressverfahren ausgebildet sein. Die optische Einheit kann demnach ebenfalls so kostengünstig hergestellt werden, so dass auf eine Wiederaufbereitung derselben verzichtet werden kann.

Das optische Element ist als eine Ophthalmoskopierlinse ausgebildet, die zur Beobachtung eines Augenhintergrundes dient. Ebenso kann das optische Element als eine Reduzierlinse ausgebildet sein, die zur Anpassung des Strahlengangs dient. Auch kann die Beobachtungsvorrichtung alleine eine Ophthalmoskopierlinse oder eine Ophthalmoskopierlinse mit einer Reduzierlinse als ein weiteres optisches Element einer weiteren optischen Einheit umfassen. So können auch zusätzliche, optische Einheiten zur Bildumkehr und/oder Vertauschung zweier Strahlengänge vorgesehen sein. Bei der Verwendung mehrerer optischer Einheiten besteht auch die Möglichkeit wieder verwertbare, sterilisierbare optische Einheiten mit nicht wieder verwertbaren optischen Einheiten aus Kunststoffmaterial an der Positioniereinheit zu kombinieren.

Weitere vorteilhafte Ausführungsformen der Beobachtungsvorrichtung ergeben sich aus den Merkmalsbeschreibungen der auf den Vorrichtungsanspruch 1 rückbezogenen Unteransprüche.

Nachfolgend werden bevorzugte Ausführungsformen der Erfindung unter Bezugnahme auf die beigefügte Zeichnung näher erläutert.

Es zeigen:
- **Fig.1**: Eine perspektivische Darstellung einer Beobachtungsvor-richtung mit einer Positioniereinheit in einer unteren Ar-beitsposition;
- **Fig.2**: eine perspektivische Darstellung der Beobachtungsvorrich-tung mit der Positioniereinheit in einer oberen Arbeitspo-sition;
- **Fig.3**: eine perspektivische Ausschnittsdarstellung einer Einstell-einrichtung;
- **Fig.4**: eine rückwärtige, perspektivische Darstellung der Beobach-tungsvorrichtung aus **Fig.1**;
- **Fig.5**: eine perspektivische Darstellung der Beobachtungsvorrich-tung aus **Fig.1** in einer Nichtverwendungsposition;
- **Fig.6**: eine perspektivische Darstellung einer weiteren Beobach-tungsvorrichtung mit einer Positioniereinheit;
- **Fig.7**: eine rückwärtige Darstellung der Beobachtungsvorrichtung aus **Fig**.**6**.

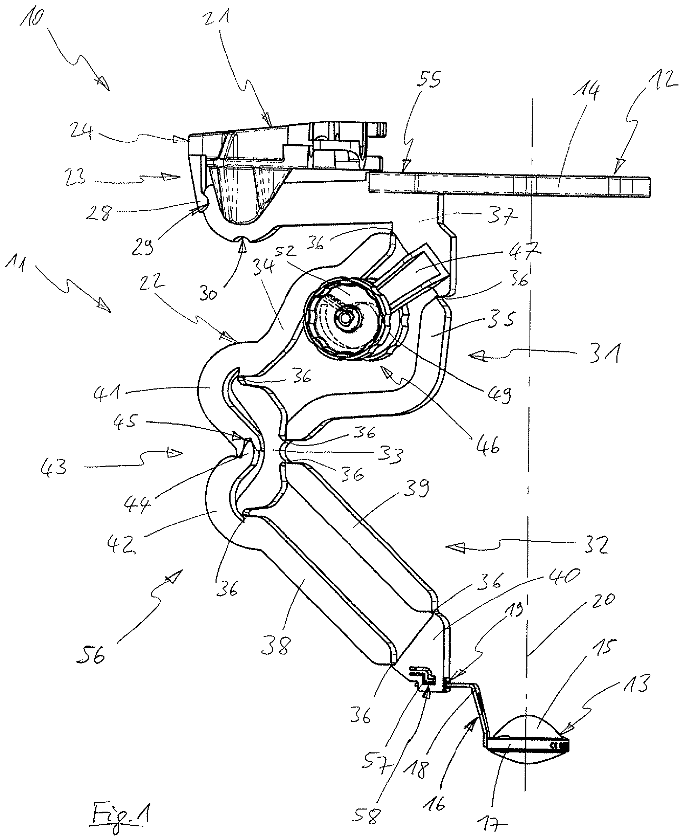

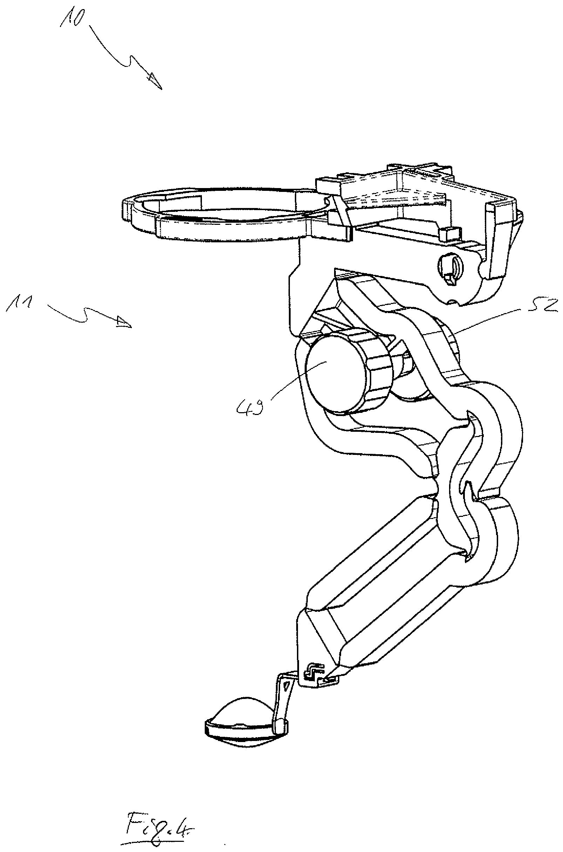

Einer Zusammenschau der **Fig.1** bis 5 ist eine Beobachtungsvorrichtung 10 mit einer Positioniereinrichtung 11 in verschiedenen Darstellungen und Positionen zu entnehmen. Die Beobachtungsvorrichtung 10 umfasst optische Einheiten 12 und 13, wobei die optische Einheit 12 hier nur teilweise dargestellt ist. Von der optischen Einheit 12 ist hier lediglich eine ringförmige Halteeinrichtung 14 zur Aufnahme einer hier nicht dargestellten Reduzierlinse gezeigt. Die optische Einheit 13 wird von einer Ophthalmoskopierlinse 15 und einer Halteeinrichtung 16 gebildet. Die Halteeinrichtung 16 umfasst eine Fassung 17 zur Halterung der Ophthalmoskopierlinse 15 und einen winkelförmigen Halter 18 zum Anschluss an eine Aufnahmevorrichtung 19 der Positioniereinrichtung 11. Die hier nicht gezeigte Reduzierlinse und die Ophthalmoskopierlinse 15, können in einem hier andeutungsweise dargestellten Strahlengang 20 eines hier nicht gezeigten Mikroskops angeordnet werden.

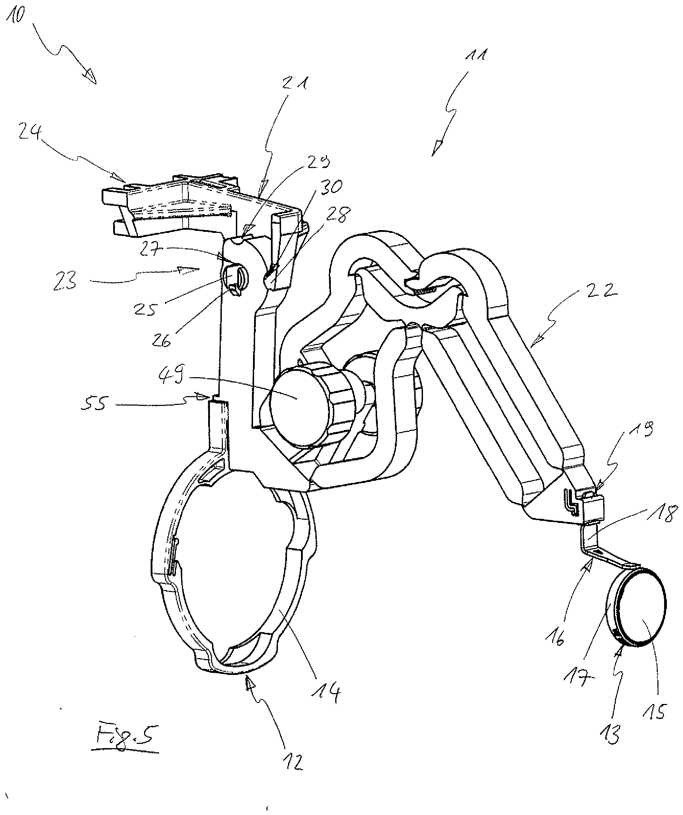

Die Positioniereinheit 11 umfasst eine Anschlussvorrichtung 21 und eine Positioniervorrichtung 22, wobei zwischen der Anschlussvorrichtung 21 und der Positioniervorrichtung 22 eine Wechselvorrichtung 23 zum Aus-und Einschwenken der Positioniervorrichtung 22 mit den optischen Einheiten 12 und 13 in den Strahlengang 20, wie aus einem Vergleich der **Fig. 1** und **5** ersichtlich ist, ausgebildet ist. Die Anschlussvorrichtung 21 besteht aus einem Kunststoffmaterial und ist durch ein Spritzgussverfahren einstückig hergestellt. An der Anschlussvorrichtung 21 sind Eingriffelemente 24 zur Verbindung der Anschlussvorrichtung 21 mit einer hier nicht dargestellten Adaptereinrichtung eines Mikroskops ausgebildet. Weiter bildet die Anschlussvorrichtung 21 eine Achse 25 mit einer Rastnase 26 aus, welche in eine Nabe 27 der Positioniervorrichtung 22 einsetzbar, und wie dargestellt verrastbar ist. Die Positioniervorrichtung 22 kann so nun um die Achse 25 verschwenkt werden. Darüber hinaus ist eine weitere Rastnase 28 an der Anschlussvorrichtung 21 ausgebildet, welche in Rastvertiefungen 29 und 30 der Positioniervorrichtung 22 einrasten kann. Die Rastvertiefungen 29 und 30 sind so an der Positioniervorrichtung 22 ausgebildet, dass die Positioniervorrichtung 22 in der **Fig. 1** dargestellten Verwendungsposition oder in der **Fig.5** dargestellten Nichtverwendungsposition durch Verrasten mit der Rastnase 28 arretierbar ist.

Die Positioniervorrichtung 22 ist aus einem ersten Doppelschwingengetriebe 31 und einem zweiten Doppelschwingengetriebe 32 gebildet. Die Doppelschwingengetriebe 31 und 32 sind mittels eines gemeinsamen Koppelgliedes 33 miteinander verbunden. Das erste Doppelschwingengetriebe weist eine Schwinge 34 und eine Schwinge 35 auf, die jeweils über Filmscharniere 36 mit dem Koppelglied 33 und mit einem Anschlusslagerglied 37 verbunden sind. Das zweite Doppelschwingengetriebe 32 weist Schwingen 38 und 39 auf, die jeweils mittels Filmscharniere 36 mit dem Koppelglied 33 und mit einem Aufnahmelagerglied 40 verbunden sind. Weiter bilden die Schwingen 34 und 38 bzw. daran ausgebildete Enden 41 und 42 ein Zahnradgetriebe 43 mit einem Zahn 44 und einem Zahnzwischenraum 45 aus. Eine Bewegung der Schwingen 34 und 35 bewirkt so eine Übertragung der Bewegung durch Abwälzen des Zahns 44 im Zahnzwischenraum 45 auf die Schwingen 38 und 39 in einem hier vorliegenden Übersetzungsverhältnis von 1:1 und somit eine Bewegung der Ophthalmoskopierlinse 15 längs des Strahlengangs 20.

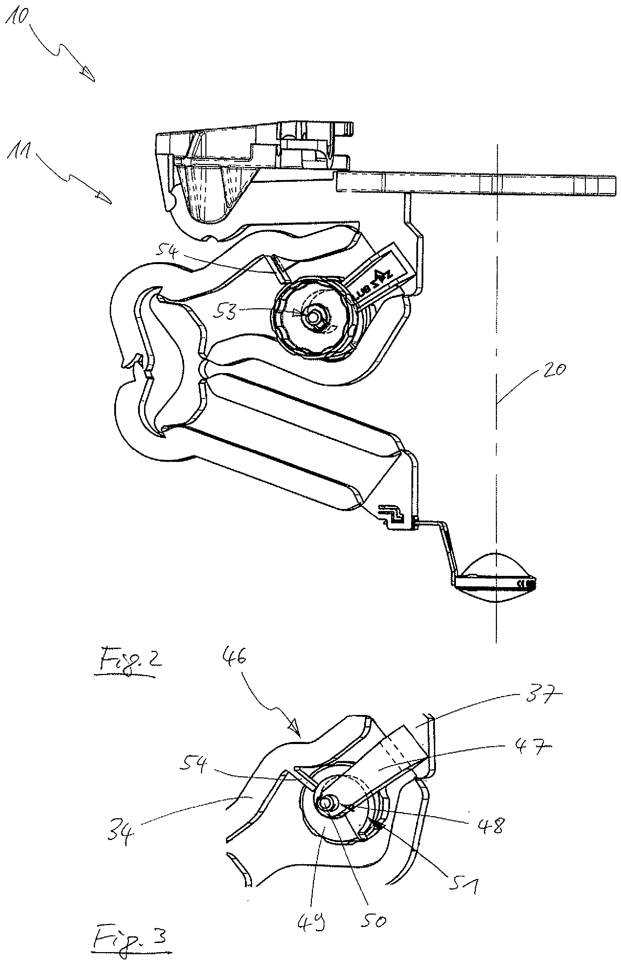

Weiter umfasst die Positioniervorrichtung 22 eine Einstelleinrichtung 46. Wie aus **Fig.3** näher ersichtlich, ist die Einstelleinrichtung 46 aus einem am Anschlusslagerglied 37 angeformten Haltelement 47 mit einer Nabe 48 gebildet. Auf die Nabe 48 ist ein Einstellrad 49 mit einer Achse 50 und einer am Einstellrad 49 angeformten Schneckenkurve 51 aufgesteckt. Gegenüber dem Einstellrad 49 ist ein weiteres Einstellrad 52 mit einer Nabe 53, wie beispielsweise aus **Fig.1** ersichtlich, auf die Achse 50 aufgesteckt. Eine Drehung der Einstellräder 49 oder 52 bewirkt nun ein Abwälzen der Schneckenkurve 51 auf einer Nocke 54, welche an die Schwinge 34 angeformt ist. Die Schwinge 34 ist so relativ zum Anschlusslagerglied 37 bewegbar, sodass mittels Drehung der Einstellräder 49 und 52 die Ophthalmoskopierlinse 15 von der in **Fig.1** gezeigten unteren Arbeitsposition in die in **Fig.2** gezeigte obere Arbeitsposition bewegt werden kann. Die Schneckenkurve 51 bzw. das Einstellrad 49 wird durch das Eigengewicht der Doppelschwingengetriebe 31 und 32 sowie der optischen Einheit 13 gegen die Nocke 54 gedrückt. Wird in Richtung des hier nicht dargestellten Mikroskops auf die Ophthalmoskopierlinse 15 eine Kraft ausgeübt, beispielsweise durch ein unbeabsichtigtes Auftreffen der Ophthalmoskopierlinse 15 auf ein zu operierendes Auge, kann die Ophthalmoskopierlinse 15, wie in **Fig.2** dargestellt, in die obere Arbeitsposition entgegen der vorgenannten Gewichtskraft bewegt werden, ohne das dazu ein größerer Kraftaufwand notwendig wäre. So kann die Nocke 54 einfach von der Schneckenkurve 51 gelöst bzw. abgehoben werden, ohne dass hierzu eine weitere Kraft aufgebracht werden müsste. Eine so ausgebildete Sicherheitseinrichtung 56 kann mögliche Verletzungen bei einer Kollision mit einem Auge wirkungsvoll verhindern.

Im Übrigen sind die Doppelschwingengetriebe 31 und 32 zusammen mit dem Halteelement 47 einstückig aus einem Kunststoffmaterial ausgebildet. Die Einstellräder 49 und 52 sind jeweils ebenfalls aus einem Kunststoffmaterial ausgebildet. So wird es möglich die Positioniereinheit 11 aus alleine vier einfach zusammensteckbaren Bauteilen aus Kunststoffmaterial herzustellen. Die Halteeinrichtung 14 ist ebenfalls aus Kunststoffmaterial gebildet, wobei die Halteeinrichtung 16 aus Metall besteht und für eine Sterilisation und Wiederverwertung vorgesehen ist. Die Positioniervorrichtung 22 umfasst weiter eine zweite Aufnahmevorrichtung 55 am Anschlusslagerglied 37 an die die ringförmige Halteeinrichtung 14 angesteckt werden kann. Alternativ ist es möglich, auch eine hier nicht gezeigte Halteeinrichtung und Ophthalmoskopierlinse aus Kunststoffmaterial zu verwenden. Um einen sicheren Halt der Halteeinrichtung 16 in der Aufnahmevorrichtung 19 zu gewährleisten, weist die in dem Aufnahmelagerglied 40 ausgebildete Aufnahmevorrichtung 19 jeweils zwei Federelemente 57 auf, die jeweils durch eine Ausnehmung 58 ausgebildet sind und hier mit nicht gezeigten Hinterschneidungen am Halter 18 verrasten können. Die Federelemente 57 drücken von oben auf den Halter 18. Die Federelemente 57 sind so ausgebildet, dass sie bei einem Entfernen des Halters 18 aus der Aufnahmevorrichtung 19 zerstört werden. Optional kann hier auch nur ein einzelnes Federelement ausgebildet sein. Somit ist für einen Benutzer ersichtlich, dass die Positioniereinheit 11 bereits verwendet wurde und nicht wiederverwertet werden kann.

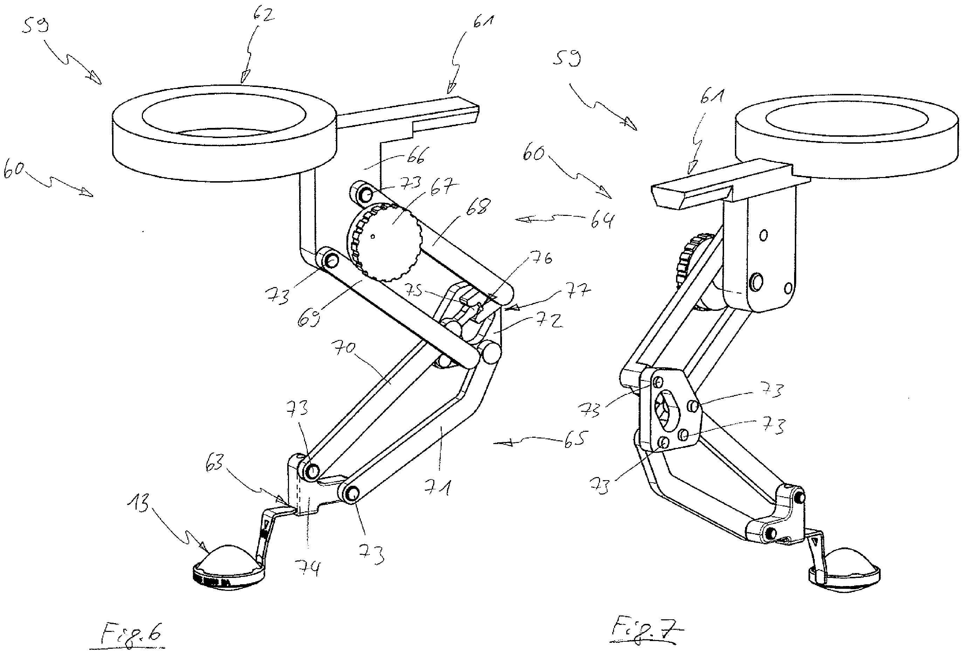

Eine Zusammenschau der **Fig.6** und 7 zeigt eine weitere Beobachtungsvorrichtung 59 mit einer Positioniereinheit 60 und einer Anschlussvorrichtung 61 an der Positioniereinheit 60. An der Positioniereinheit 60 ist eine Halteeinrichtung 62 zur Aufnahme einer hier nicht gezeigten Reduzierlinse ausgebildet. Weiter ist an einer Anschlussvorrichtung 63 der Positioniereinheit 60 eine, wie in den **Fig.1** bis **5** dargestellte optische Einheit 13 angesteckt. Im Unterschied zu der in den **Fig.1** bis **5** dargestellten Positioniereinheit sind hier ein erstes Doppelschwingengetriebe 64 und ein zweites Doppelschwingengetriebe 65 mehrteilig ausgebildet. Das erste Doppelschwingengetriebe 64 umfasst ein Anschlusslagerglied 66 an dem ein Einstellrad 67 drehbar gelagert ist und Schwingen 68 und 69, die wie Schwingen 70 und 71 des zweiten Doppelschwingengetriebes 65 an einem Koppelglied 72 über jeweils Stiftverbindungen 73 drehbar gelagert sind. Die Schwingen 70 und 71 sind mit einem Aufnahmelagerglied ebenfalls über die Stiftverbindungen 73 drehbar verbunden. An der Schwinge 70 ist ein Zahn 75 ausgebildet, der in einen Zahnzwischenraum 76 der Schwinge 68 eingreift und so ein Zahnradgetriebe 77 ausbildet.
Eine Funktion der Doppelschwingengetriebe 64 und 65 mit dem Einstellrad 67 entspricht im Wesentlichen der zuvor beschriebenen Funktion der Positioniereinheit aus den **Fig.1** bis **5**. Die Positioniereinheit 60 kann aufgrund der einfachen Form der Bauteile leicht auch aus Metall kostengünstig ausgebildet werden, wobei auch hier die Positioniereinheit 60 in einem überwiegenden Teil aus Kunststoffmaterial gebildet ist.

## Patentansprüche

1. Mikroskop, umfassend eine Beobachtungsvorrichtung (10, 59) mit einer Positioniereinheit (11, 60) und zumindest einer optischen Einheit (12, 13), wobei die optische Einheit zumindest eine Ophthalmoskopierlinse (15) umfasst, wobei die Positioniereinheit zur Positionierung der Ophthalmoskopierlinse in einem Strahlengang (20) des Mikroskops zwischen einem Objektiv des Mikroskops und vor einem zu beobachtenden Auge dient, wobei die Positioniereinheit eine Anschlussvorrichtung (21, 61) umfasst, mittels der die Positioniereinheit mit dem Mikroskop fest verbunden ist, wobei die Positioniereinheit eine Positioniervorrichtung (22) umfasst, mittels der die Ophthalmoskopierlinse relativ zum Mikroskop in Längsrichtung des Strahlengangs bewegbar ist,
**dadurch gekennzeichnet,**
**dass** die Positioniervorrichtung aus einem ersten Doppelschwingengetriebe (31, 64) und einem zweiten Doppelschwingengetriebe (32, 65) gebildet ist, wobei die Doppelschwingengetriebe mittels eines gemeinsamen Koppelgliedes (33, 72) miteinander verbunden sind, wobei das erste Doppelschwingengetriebe (31, 64) mit dem zweiten Doppelschwingengetriebe (32, 65) über ein Zahnradgetriebe (43, 72) verbunden ist, derart, dass eine Bewegung des ersten Doppelschwingengetriebes auf das zweite Doppelschwingengetriebe übertragbar ist, derart, dass die Ophthalmoskopierlinse relativ zum Mikroskop in Längsrichtung des Strahlengangs bewegbar ist.

2. Mikroskop nach Anspruch 1,
**dadurch gekennzeichnet,**
**dass** die Positioniereinheit (11, 60) zumindest teilweise aus Kunststoffmaterial gebildet ist.

3. Mikroskop nach Anspruch 1 oder 2,
**dadurch gekennzeichnet,**
**dass** die Doppelschwingengetriebe (31, 32) und das Zahnradgetriebe (43) zusammen einstückig ausgebildet sind.

4. Mikroskop nach einem der vorangehenden Ansprüche,
**dadurch gekennzeichnet,**
**dass** das Zahnradgetriebe (43, 77) ein Übersetzungsverhältnis von 1:1 aufweist.

5. Mikroskop nach einem der vorangehenden Ansprüche,
**dadurch gekennzeichnet,**
**dass** eine erste Doppelschwinge des ersten Doppelschwingengetriebes (31, 64) an einem Anschlusslagerglied (37, 66) und eine zweite Doppelschwinge des zweiten Doppelschwingengetriebes (32, 65) an einem Aufnahmelagerglied (40, 74) gelagert ist.

6. Mikroskop nach einem der vorangehenden Ansprüche,
**dadurch gekennzeichnet,**
**dass** die Positioniervorrichtung (22) eine Einstelleinrichtung (46) umfasst, mittels der eine Position der Ophthalmoskopierlinse (15) einstellbar ist.

7. Mikroskop nach Anspruch 6,
**dadurch gekennzeichnet,**
**dass** die Einstelleinrichtung (46) aus zumindest einem Einstellrad (40, 52, 67) mit einem Schnecken- oder Exzentergetriebe (51) gebildet ist.

8. Mikroskop nach einem der vorangehenden Ansprüche,
**dadurch gekennzeichnet,**
**dass** die Positioniervorrichtung (22) eine Sicherheitseinrichtung (56) ausbildet, die eine lose Bewegung der Ophthalmoskopierlinse (15) ermöglicht, wenn auf die Ophthalmoskopierlinse eine Kraft in Richtung des Mikroskops ausgeübt wird.

9. Mikroskop nach einem der vorangehenden Ansprüche,
**dadurch gekennzeichnet,**
**dass** die Positioniereinheit (11) eine Wechselvorrichtung (23) umfasst, mittels der die Ophthalmoskopierlinse (15) in den Strahlengang (20) hinein und heraus bewegbar ist.

10. Mikroskop nach Anspruch 9,
**dadurch gekennzeichnet,**
**dass** die Wechselvorrichtung (23) durch die Anschlussvorrichtung (21) und die Positioniervorrichtung (22) ausgebildet ist, derart, dass die Positioniervorrichtung relativ zur Anschlussvorrichtung verschwenkbar ist.

11. Mikroskop nach Anspruch 9 oder 10,
**dadurch gekennzeichnet,**
**dass** die Wechselvorrichtung (23) als ein Drehgelenk ausgebildet ist, wobei die Wechselvorrichtung zumindest eine Rasteinrichtung (28, 29) umfasst, mittels der die Ophthalmoskopierlinse (15) in einer Verwendungsposition im Strahlengang (22) und/oder in einer Nichtverwendungsposition außerhalb des Strahlengangs arretierbar ist.

12. Mikroskop nach einem der vorangehenden Ansprüche,
**dadurch gekennzeichnet,**
**dass** Schwingenlager der Doppelschwingengetriebe (31, 32) durch jeweils ein Filmscharnier (36) ausgebildet sind.

13. Mikroskop nach einem der vorangehenden Ansprüche,
**dadurch gekennzeichnet,**
**dass** die Positioniereinheit (11) vollständig aus Kunststoffmaterial ausgebildet ist.

14. Mikroskop nach einem der vorangehenden Ansprüche,
**dadurch gekennzeichnet,**
**dass** die Positioniereinheit (11, 60) eine Aufnahmevorrichtung (19, 55, 63) umfasst, mittels der die optische Einheit an der Positioniereinheit adaptierbar ist, wobei die optische Einheit eine Halteeinrichtung (16) zur Halterung und Verbindung der Ophthalmoskopierlinse (15) mit der Aufnahmevorrichtung ausbildet.

15. Mikroskop nach einem der Ansprüche 1 bis 13,
**dadurch gekennzeichnet,**
**dass** die Positioniereinheit (60) eine Halteeinrichtung (62) zur Halterung der Ophthalmoskopierlinse (15) ausbildet.

16. Mikroskop nach einem der Ansprüche 14 oder 15,
**dadurch gekennzeichnet,**
**dass** die Halteeinrichtung (16) und/oder die Aufnahmevorrichtung (19, 55, 63) und/oder eine Anschlussvorrichtung (21, 61) zumindest ein Verbindungselement (57) aufweist, welches so ausgebildet ist, dass es bei einem Trennen von Halteeinrichtung und Aufnahmevorrichtung und/oder Anschlussvorrichtung und einem Mikroskop zerstört wird.

## Claims

1. A microscope, comprising a monitoring device (10, 59) having a positioning unit (11, 60) and at least one optical unit (12, 13), the optical unit comprising at least one ophthalmoscopic lens (15), the positioning unit serving to position the ophthalmoscopic lens in a beam path (20) of the microscope between an objective lens of the microscope and in front of an eye to be monitored, the positioning unit comprising a connection device (21, 61), by means of which the positioning unit is securely connected to the microscope, the positioning unit comprising a positioning device (22), by means of which the ophthalmoscopic lens is movable relative to the microscope in the longitudinal direction of the beam path,
**characterised in that**
the positioning device is formed of a first double rocker mechanism (31, 64) and a second double rocker mechanism (32, 65), the double rocker mechanisms being interconnected by means of a common coupling member (33, 72), the first double rocker mechanism (31, 64) being connected to the second double rocker mechanism (32, 65) via a toothed gearing (43, 72) in such a way that a movement of the first double rocker mechanism is transferrable to the second double rocker mechanism in such a way that the ophthalmoscopic lens is movable relative to the microscope in the longitudinal direction of the beam path.

2. The microscope according to claim 1,
**characterised in that**
the positioning unit (11, 60) is formed, at least in part, of plastics material.

3. The microscope according to claim 1 or 2,
**characterised in that**
the double rocker mechanisms (31, 32) and the toothed gearing (43) are formed together in one piece.

4. The microscope according to any one of the preceding claims,
**characterised in that**
the toothed gearing (43, 77) has a gear ratio of 1:1.

5. The microscope according to any one of the preceding claims,
**characterised in that**
a first double rocker of the first double rocker mechanism (31, 64) is mounted on a connecting bearing member (37, 66), and a second double rocker of the second double rocker mechanism (32, 65) is mounted on a receiving bearing member (40, 74).

6. The microscope according to any one of the preceding claims,
**characterised in that**
the positioning device (22) comprises an adjustment means (46), by means of which a position of the ophthalmoscopic lens (15) is adjustable.

7. The microscope according to claim 6,
**characterised in that**
the adjustment means (46) is formed of at least one adjusting wheel (40, 52, 67) having a worm gear or an eccentric gear (51).

8. The microscope according to any one of the preceding claims,
**characterised in that**
the positioning device (22) forms a safety means (56) which allows a loose movement of the ophthalmoscopic lens (15) if a force is exerted on the ophthalmoscopic lens in the direction of the microscope.

9. The microscope according to any one of the preceding claims,
**characterised in that**
the positioning unit (11) comprises an alternating device (23), by means of which the ophthalmoscopic lens (15) can be moved into and out of the beam path (20).

10. The microscope according to claim 9,
**characterised in that**
the alternating device (23) is formed by the connection device (21) and the positioning device (22) in such a way that the positioning device is pivotable relative to the connection device.

11. The microscope according to either claim 9 or claim 10,
**characterised in that**
the alternating device (23) is formed as a hinge, the alternating device comprising at least one snap-in means (28, 29), by means of which the ophthalmoscopic lens (15) is lockable in a use position in the beam path (22) and/or in an idle position outside the beam path.

12. The microscope according to any one of the preceding claims,
**characterised in that**
link brackets of the double rocker mechanisms (31, 32) are formed in each case by a living hinge (36).

13. The microscope according to any one of the preceding claims,
**characterised in that**
the positioning unit (11) is formed completely of plastics material.

14. The microscope according to any one of the preceding claims,
**characterised in that**
the positioning unit (11, 60) comprises a receiving device (19, 55, 63), by means of which the optical unit can be adapted to the positioning unit, the optical unit forming a holding means (16) for holding the ophthalmoscopic lens (15) and connecting it to the receiving device.

15. The microscope according to any one of claims 1 to 13,
**characterised in that**
the positioning unit (60) forms a holding means (62) for holding the ophthalmoscopic lens (15).

16. The microscope according to either claim 14 or claim 15,
**characterised in that**
the holding means (16) and/or the receiving device (19, 55, 63) and/or a connection device (21, 61) comprises at least one connecting element (57) which is formed in such a way that it is destroyed upon separation of the holding means and receiving device and/or connection device and a microscope.

## Revendications

1. Microscope, comprenant un dispositif de surveillance (10, 59) ayant une unité de positionnement (11, 60) et au moins une unité optique (12, 13), l'unité optique comprenant au moins une lentille ophtalmoscopique (15), l'unité de positionnement servant à positionner la lentille ophtalmoscopique dans un trajet optique (20) du microscope entre un objectif du microscope et devant un oeil à surveiller, l'unité de positionnement comprenant un dispositif de connexion (21, 61), par l'intermédiaire duquel l'unité de positionnement est solidaire du microscope, l'unité de positionnement comprenant un dispositif de positionnement (22), par l'intermédiaire duquel la lentille ophtalmoscopique est déplaçable par rapport au microscope dans la direction longitudinale du trajet optique,
**caractérisé en ce que**
le dispositif de positionnement est formé par un premier mécanisme à deux bielles (31, 64) et un deuxième mécanisme à deux bielles (32, 65), les mécanismes à deux bielles étant interconnectés par un organe d'accouplement (33, 72) commun, le premier mécanisme à deux bielles (31, 64) étant connecté au deuxième mécanisme à deux bielles (32, 65) par un engrenage (43, 72) de telle manière qu'un mouvement du premier mécanisme à deux bielles est transférable au deuxième mécanisme à deux bielles de telle manière que la lentille ophtalmoscopique est déplaçable par rapport au microscope dans la direction longitudinale du trajet optique.

2. Microscope selon la revendication 1,
**caractérisé en ce que**
l'unité de positionnement (11, 60) est fabriqué, au moins en partie, en matériau plastique.

3. Microscope selon la revendication 1 ou 2,
**caractérisé en ce que**
les mécanismes à deux bielles (31, 32) et l'engrenage (43) sont formés ensemble d'un seul tenant.

4. Microscope selon l'une quelconque des revendications précédentes,
**caractérisé en ce que**
l'engrenage (43, 77) a un rapport de transmission de 1:1.

5. Microscope selon l'une quelconque des revendications précédentes,
**caractérisé en ce qu'**
une première bielle double du premier mécanisme à deux bielles (31, 64) est montée sur un organe palier de connexion (37, 66) et une deuxième bielle double du deuxième mécanisme à deux bielles (32, 65) est montée sur un organe palier de logement (40, 74).

6. Microscope selon l'une quelconque des revendications précédentes,
**caractérisé en ce que**
le dispositif de positionnement (22) comprend un moyen d'ajustement (46) à l'aide duquel une position de la lentille ophtalmoscopique (15) est ajustable.

7. Microscope selon la revendication 6,
**caractérisé en ce que**
le moyen d'ajustement (46) est formé par au moins une roue d'ajustement (40, 52, 67) avec un engrenage à vis sans fin ou excentrique (51).

8. Microscope selon l'une quelconque des revendications précédentes,
**caractérisé en ce que**
le dispositif de positionnement (22) forme un moyen de sécurité (56) qui permet un déplacement lâche de la lentille ophtalmoscopique (15) quand une force est exercée sur la lentille ophtalmoscopique dans la direction du microscope.

9. Microscope selon l'une quelconque des revendications précédentes,
**caractérisé en ce que**
l'unité de positionnement (11) comprend und dispositif de changement (23) à l'aide duquel la lentille ophtalmoscopique (15) peut être rentrée dans le trajet optique (20) et sortie du trajet optique.

10. Microscope selon la revendication 9,
**caractérisé en ce que**
le dispositif de changement (23) est formé par le dispositif de connexion (21) et le dispositif de positionnement (22) de telle manière que le dispositif de positionnement est pivotable par rapport au dispositif de connexion.

11. Microscope selon la revendication 9 ou 10,
**caractérisé en ce que**
le dispositif de changement (23) est formé comme articulation tournante, le dispositif de changement comprenant au moins un moyen d'arrêt (28, 29), à l'aide duquel la lentille ophtalmoscopique (15) peut être arrêtée dans une position d'utilisation dans le trajet optique (22) et/ou dans une position de non-utilisation en dehors du trajet optique.

12. Microscope selon l'une quelconque des revendications précédentes,
**caractérisé en ce que**
des paliers de bielle des mécanismes à deux bielles (31, 32) sont formés chacun par une charnière souple (36).

13. Microscope selon l'une quelconque des revendications précédentes,
**caractérisé en ce que**
l'unité de positionnement (11) est formée entièrement en matériau plastique.

14. Microscope selon l'une quelconque des revendications précédentes,
**caractérisé en ce que**
l'unité de positionnement (11, 60) comprend un dispositif de logement (19, 55, 63), à l'aide duquel l'unité optique peut être adaptée à l'unité de positionnement, l'unité optique formant un moyen de support (16) destiné a supporter la lentille ophtalmoscopique (15) et à connecter la lentille ophtalmoscopique au dispositif de logement.

15. Microscope selon l'une quelconque des revendications 1 à 13,
**caractérisé en ce que**
l'unité de positionnement (60) forme un moyen de support (62) destiné à supporter la lentille ophtalmoscopique (15).

16. Microscope selon la revendication 14 ou 15,
**caractérisé en ce que**
le moyen de support (16) et/ou le dispositif de logement (19, 55, 63) et/ou un dispositif de connexion (21, 61) comprend au moins un élément de liaison (57) qui est formé de telle manière qu'il est détruit lors d'une séparation du moyen de support et du dispositif de logement et/ou du dispositif de connexion et d'un microscope.
